# EUROPEAN PATENT APPLICATION

(11) **EP 2 617 354 A1**
(43) Date of publication of application: **24.07.2013**
(21) Application number: 12197491.9
(22) Date of filing: 17.12.2012
(51) Int. Cl.: A61B 5/00, G11B 27/00, H04N 21/422

(54) **Camera button with integrated sensors**

(30) Priority: 17.01.2012 US 201261587158 P; 15.11.2012 US 201213677517
(71) Applicant: Sony Mobile Communications AB, 221 88 Lund (SE)
(72) Inventor: Klinghult, Lennart, 223 59 Lund (SE)
(74) Representative: Östlund, Johanna

(57) **Abstract**

The present invention relates to a method and a communication device for tagging a recorded image in a mobile communication device. The recorded image is recorded by a camera unit in the mobile communication device. The method comprising the steps of monitoring, using at least one sensor in the mobile communication device, a user's vital signs, recording an image and sensor information relating to the user's vital signs when the user operates the camera unit, determining a tag based on the recorded sensor information, and assigning the tag to the recorded image and storing the recorded image in a memory in the mobile communication device based on the tag.

## Description

### TECHNICAL FIELD

The invention relates in general to the field of mobile communication devices fitted with camera units, and particularly to the tagging of images taken with the camera units in the mobile communication devices.

### BACKGROUND

Today's mobile communication devices are often fitted with a camera unit. The camera unit, together with large storage capabilities, has made the mobile communication device one of people's favourite devices for taking photos and shooting videos with. With an ever increasing amount of photos and videos stored in the mobile phone, in the cloud or at the home computer it becomes more and more difficult to categorize and organize the photos and movies. The traditional way of tagging files to facilitate organization of them is in most cases rather impersonal (nearly always focusing on a particular event e.g. "Christmas dinner with family") and takes too much time and effort. Finding a way to facilitate the tagging process of photos and movies in a mobile communication device, and make the tagging and organization of them more personal, is thus highly sought after.

### SUMMARY OF THE INVENTION

With the above description in mind, then, an aspect of the present invention is to provide a way to facilitate the tagging of photos and movies taken with a mobile communication device, and make the tagging and organization of them more personal, which seeks to mitigate, alleviate, or eliminate one or more of the above-identified deficiencies in the art and disadvantages singly or in any combination.

As will be described in more detail by the aspects of the present invention below, one way to make the tags more personal is to integrate sensors in the mobile communication device which may provide sensor information about the user that may be used in an automatic tagging of images when taken, according to the aspects of the present invention below.

A first aspect of the present invention relates to a method for tagging a recorded image in a mobile communication device, wherein said recorded image is recorded by a camera unit in said mobile communication device, the method comprising the steps monitoring, using at least one sensor in said mobile communication device, a user's vital signs, recording sensor information relating to said user's vital signs when said user operates said camera unit in said mobile communication device and is in contact with at least one of said at least one sensors in said mobile communication device, recording an image from said camera unit when said user operates said camera unit in said mobile communication device, determining a tag based on said recorded sensor information, assigning said tag to said recorded image and storing and organizing said recorded image in a memory in said mobile communication device based on said tag.

The method wherein said sensor information may comprise information regarding any of the following user's vital signs: body temperature, pulse rate, blood pressure, respiratory rate, blood oxygen level and skin conductance.

The method wherein at least one of said at least one sensor may be integrated in a camera button in said mobile communication device, wherein said sensor and camera button may be operated when said user operates said camera unit for recording an image.

The method wherein an image may be any of a photograph and a movie.

The method wherein said at least one sensor may be any of: an optical pulse rate sensor, a blood oxygen sensor, an accelerometer, a temperature sensor, and a sensor for measuring electrical resistance.

The method may further comprise recording sensor information relating to said user's activity and position from activity sensors and positioning sensors in said mobile communication device, and wherein said determining of said tag may further be based on said recorded sensor information relating to said user's activity and position.

The method wherein said at least one sensor may be placed in a position on the casing of the mobile communication device where said user may hold at least one body part when operating said mobile communication device, and wherein said monitoring, using at least one sensor, of said user's vital signs may be performed via at least one of said at least one body part.

A second aspect of the present invention relates to a mobile communication device adapted for tagging a recorded image, the mobile communication device comprising a camera unit adapted to record an image, at least one sensor adapted for monitoring a user's vital signs, processing means configured to, monitoring, using said at least one sensor, a user's vital signs when said user is in contact with at least one of said at least one sensor, recording sensor information relating to said user's vital signs when said user operates said camera unit, recording an image from said camera unit when said user operates said camera unit in said mobile communication device, determining a tag based on said recorded sensor information, assigning said tag to said recorded image, and storing means and organizing means adapted to store and to organize said recorded image in a memory in said mobile communication device based on said tag.

The mobile communication device wherein said sensor may be adapted to monitor sensor information relating to any of the following user's vital signs: body temperature, pulse rate, blood pressure, blood oxygen level, respiratory rate, and skin conductance.

The mobile communication device wherein at least one of said at least one sensor may be integrated in a camera button in said mobile communication device, wherein said sensor and camera button may be operated when said user operates said camera unit for recording an image.

The mobile communication device wherein said recorded image may be any of a photograph and a movie.

The mobile communication device wherein said at least one sensor may be any of: an optical pulse rate sensor, a temperature sensor, a blood oxygen sensor, an accelerometer, and a sensor for measuring electrical resistance.

The mobile communication device may further comprise at least one activity sensor adapted to record sensor information relating to said user's activity, at least one positioning sensor adapted to record the position of said mobile communication device, wherein said processing means may further be adapted to further determine said tag based on said recorded sensor information relating to said user's activity and to the position of said mobile communication device.

The mobile communication device wherein said at least one sensor may be placed in a position on the casing of the mobile communication device where said user holds at least one body part when operating said mobile communication device, and wherein said monitoring, using at least one sensor, of said user's vital signs may be performed via at least one of said at least one body part.

The variants presented in conjunction with the first and the second aspect of the present invention described above may be combined in any way possible to form different variants and or embodiments of the present invention.

### BRIEF DESCRIPTION OF THE DRAWINGS

Further objects, features, and advantages of the present invention will appear from the following detailed description of some embodiments of the invention, wherein some embodiments of the invention will be described in more detail with reference to the accompanying drawings, in which:
Fig. 1 a shows the front side of a mobile phone with several sensor areas indicated, according to an embodiment of the present invention;
Fig. 1b the back side of a mobile phone with several sensor areas indicated, according to an embodiment of the present invention;
Fig. 2a shows an exploding view of a typical optical pulse rate sensor, according to an embodiment of the present invention;
Fig. 2b shows a camera trigger button with an integrated sensor, according to the present invention;
Fig. 3 shows a flowchart describing a method according to the present invention; and
Fig. 4 shows a block diagram of the mobile communication device according to an embodiment of the present invention.

### DETAILED DESCRIPTION

Embodiments of the present invention will be described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art.

Embodiments of the present invention will be exemplified using a mobile phone with a built in camera unit. However, it should be appreciated that the invention is as such equally applicable to any type of pocket sized mobile communication device with a built in camera unit. Examples of such devices may for instance be any type of hand-held navigation devices, handheld computers, portable digital assistants, tablets and pads, gaming devices, accessories to mobile phones, etc. However, for the sake of clarity and simplicity, the embodiments outlined in this specification are exemplified with and related to mobile phones with a built in camera unit.

One way of improving the tagging and organization of photos and movies (hereinafter collectively referred to as images) recorded with a mobile communication device with a built in camera unit, and making the tags more personal, is to automatically tag each image with information reflecting the current state of the user's body, such as the current levels of excitement, tension, anxiety, relaxation, work load, temperature, etc. Not only will this provide a more elaborate and personal tagging of the recorded images than any conventional way of "tagging", such as manually renaming the image, but it will also provide a new and more personal dimension to the organization of the images since it may be possible to organize them after the state that the user (or the state of his or hers body) was in at the moment when the image was recorded.

The term image refers to, and should be interpreted as, any type of 2-dimensional or 3-dimensional still image or a 2-dimensional or 3-dimensional kinetic image (also called a moving image or a movie).

A user's vital signs provide the information need to determine the current state of a user's body. Vital signs are measures of various physiological statistics in order to assess the state of a person's body functions. The act of taking vital signs normally entails recording one or several of the parameters;
- temperature,
- pulse rate (or heart rate),
- blood pressure, and
- respiratory rate,
but may also include measuring other parameters such as the galvanic skin response and the blood oxygen level. Usually when measuring vital signs, sensors, connected by wires to bulky measuring equipment, have to be placed or used on the person's body. However, in this case the measuring of vital signs has to be done in another way since using cumbersome sensors and measuring equipment is not a viable option.

According to the present invention one way of measuring the vital signs of a user without the need of bulky equipment is to integrate sensors capable of monitoring and recording the user's vital signs into the mobile phone. The user's vital signs may then be monitored and recorded when he or she is operating the mobile phone and recording images.

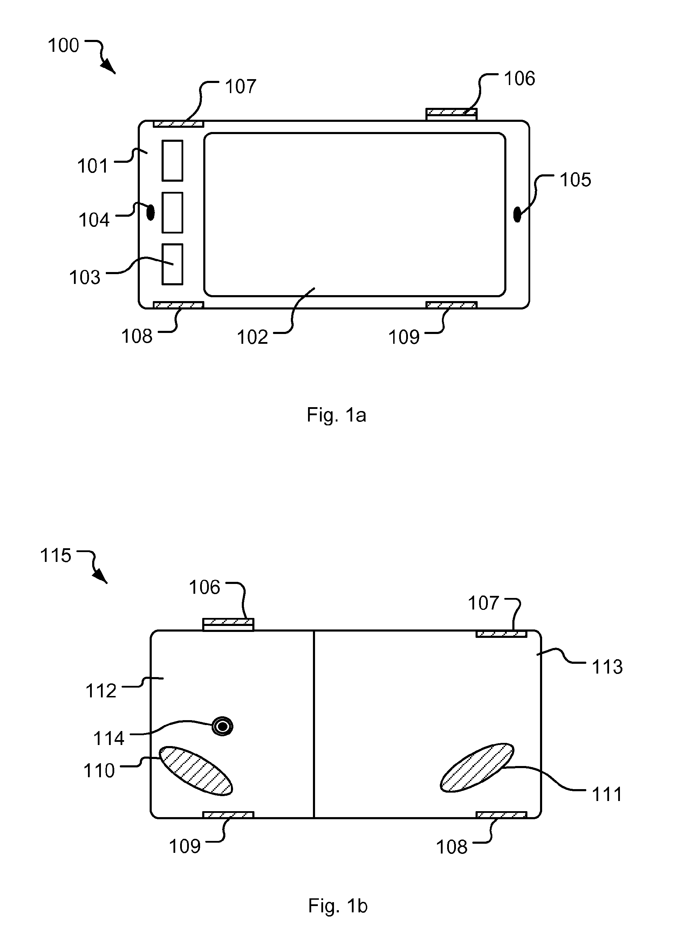

According to an embodiment of the present invention at least one sensor may be placed at at least one key position on the casing of the mobile phone to monitor the vital signs of the user of the mobile communication device. Figure 1 a shows the front 100 of a typical mobile phone comprising a casing 101, a display area 102, navigational means 103 (e.g. buttons), a microphone opening 104 and a loudspeaker opening 105. The stripped areas 106,107,108,109 on the mobile phone indicates preferred key areas wherein one or more sensors for monitoring and recording the user's vital signs may be placed.

These areas 106,107,108,109 are areas in which it is very likely that the user of the mobile phone places one or more fingers at when recording an image with the camera unit (not shown in figure 1 a). Usually, when a user have activated the camera in the mobile phone, by for instance operating the navigation means 103 (i.e. one of the buttons on the mobile phone), and is pointing the camera unit at the object of interest, the user usually holds the mobile phone in a certain way. If the user is right-handed the user will at least place one finger at the camera button 106 (which is the primary key position in which to place a sensor), usually the distal phalanx of the index finger, and another finger, usually the distal phalanx of the thumb, as a support on the other side of the mobile phone casing 109 to be able to push down with the index finger. Thus, by placing at least one sensor in the vicinity of (or in) the camera button 106, it is possible to gather sensor information such as the user's vital signs when the user is in the process of recording an image.

When recording an image, the user would usually try to further stabilize the camera by grabbing it at the other end (compared to the end with the camera button 106) with his or hers left hand fingers (i.e. the distal phalanx of the index finger and the distal phalanx of the thumb) in a similar manner as with his or hers right hand fingers. Thus, sensors aiding in determining the user's vital signs may also be placed at these key locations 107,108 at the other end of the mobile phone in relation to the end of the mobile phone with the camera button.

Figure 1b shows the back 115 of the same mobile phone as in figure 1a. The back side 115 comprise a first back casing 112 comprising a camera unit 114, and a second back casing 113, which may be removable to expose the mobile phone batteries. The back side 115 of the mobile phone also comprise to additional sensor areas 110,111. It is common that the user grabbing the camera with the index finger and the thumb, with one or with both hands, often bends his middle finger and places it on the back side casing (in relation to the side with the display) as additional support. Thus, additional sensors for aiding in determining the user's vital signs may be placed in these key areas. Additional sensors may be placed in other areas of the mobile phone and the shape, form, and size of the sensors may vary from that shown in figure 1a and 1b. Also more than one sensor may be placed in each area shown in figures 1 a and 1 b.

The sensors 106,107,108,109,110,111 in the mobile phone shown in figure 1 a and 1 b may be implemented in different ways depending on what they are suppose to measure. The sensors 106,107,108,109,110, 111 may for instance be implemented as;
thermistors or thermocouples, for measuring the temperature of the body part (for example the users fingers) of the user that comes in contact with the sensor,
conducting electrodes in a 2-lead electrocardiogram (ECG) measuring system for measuring the users hart rate,
conducting electrodes in a galvanic skin response measurement system for determining the electrical conductance of the skin which may be used as an indication of psychological or physiological arousal,
pressure sensors, for determining the pulse rate or the blood pressure via the pulse pressure,
trigger sensors, (on - when touched, off - when not touched) for activating one or more accelerometers in a system for determining the user's respiratory rate, or
optical sensors, for determining the pulse rate and/or the blood oxygen level. However, if for instance the pulse rate, the temperature, the respiratory rate, or the blood oxygen level is to be determined, only one of the sensors 106,107,108,109,110,111 in the mobile phone has to be implemented. If only one sensor is to be used then preferably the sensor in the camera button 106 is chosen since this button always is pressed when recording an image. To get a more accurate reading, more than one sensor measuring the same quantity may be implemented so that an average (and more accurate) measured value may be determined. If the heart rate or the galvanic skin response of the user is going to be measured at least two sensors, or electrodes, must be implemented.

If the galvanic skin response is to be measured it may be wise to choose to implement the two sensors at locations 106,107,108,109,110,111 in the mobile phone where it is most likely that a user would touch the sensors. In this case the camera button 106 and the location opposite to the camera button 109 or at the other end of the mobile phone at the locations 107 and 108 may preferably be chosen.

The heart rate is best measured through the body, which in this case means from one hand to the other hand. In this case the camera button 106 and the location 107 at the other end of the mobile phone or the camera button 106 and the location 108 at the opposite side at the other end of the mobile phone may preferably be chosen.

Other combinations may also be chosen but the described above are the considered to be the preferred locations to implement sensors in depending on which quantity that is going to be measured.

The sensor 106 or the sensors 106,107,108,109,110,111 may constantly or in intervals monitor the user's vital signs as long as the user is in physical contact (with a body part, preferably his or hers fingers) with one or more of the sensors 106,107,108,109,110,111 implemented in the mobile phone. The monitoring may begin when the camera unit and/or camera application in the mobile phone is activated. When the user records an image i.e. takes a photo the vital signs, which may be the vital signs at the moment when the camera button is pressed or the vital signs from a time interval before the camera button is pressed (or even a time interval after the camera button is pressed), registered by the at least one sensor is recorded. A processor 409 in the mobile phone may then process the recorded sensor information and determine a tag based on said recorded sensor information.

The process of determining the tag may be user defined or it may be preset from the factory or a combination of the both. For example, the user may have indicated in a user interface in the mobile phone that he or she wants to record his or hers pulse rate with every photo. Then only sensor information relating to pulse rate is gathered from the user by the sensors, and a tag with the information "current pulse rate" is determined. In another example it is factory preset that the user's general fitness should be tagged with each movie. Thus, when the user records an image comprising a movie, sensor information relating to body temperature, pulse rate, blood pressure and respiratory rate are recorded and a general "fitness index tag" is determined using a special algorithm wherein the sensor information is used to calculate a fitness index.

The determined tag is then assigned to the tag and stored in a memory 410 in the mobile phone. The tag may then be used to organize and sort all the images taken by the user. In a variant both the tag and the "raw" sensor information is stored together with the image, while in another variant only the determined tag is stored with the image.

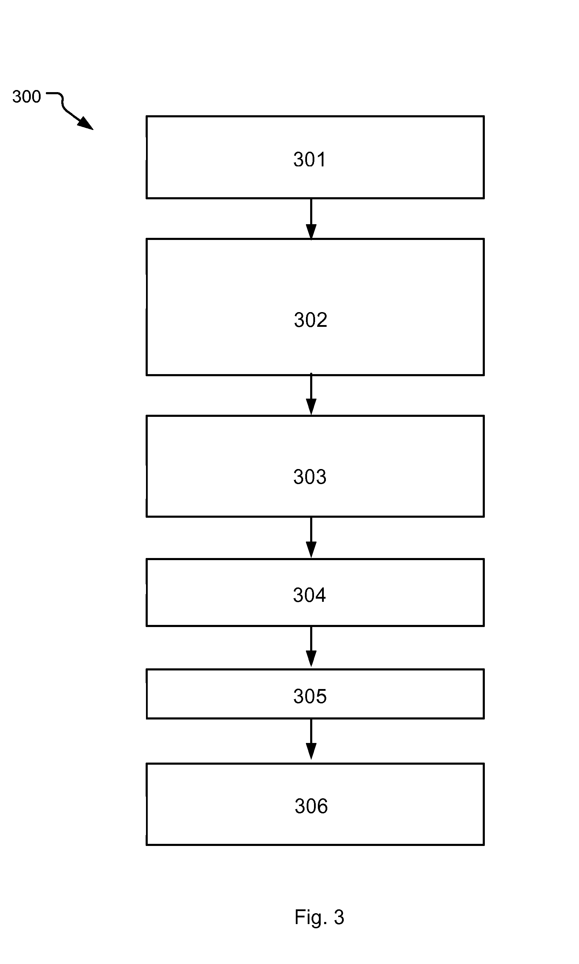

The method for tagging a recorded image in a mobile communication device may be broken down into a series of steps as shown in a flowchart in figure 3. The main steps of the method are:
I) Monitoring 301, using at least one sensor in the mobile communication device, a user's vital signs.
II) Recording sensor information 302 relating to the user's vital signs when the user operates the camera unit in the mobile communication device.
III) Recording an image 303 from the camera unit when the user operates the camera unit in the mobile communication device.
IV) Determining a tag 304 based on the recorded sensor information.
V) Assigning the tag 305 to the recorded image.
VI) Storing 306 the recorded image in a memory in the mobile communication device based on the tag.

This method will thus provide a new and personal way of tagging and organizing recorded images in comparison with the traditional way of tagging images.

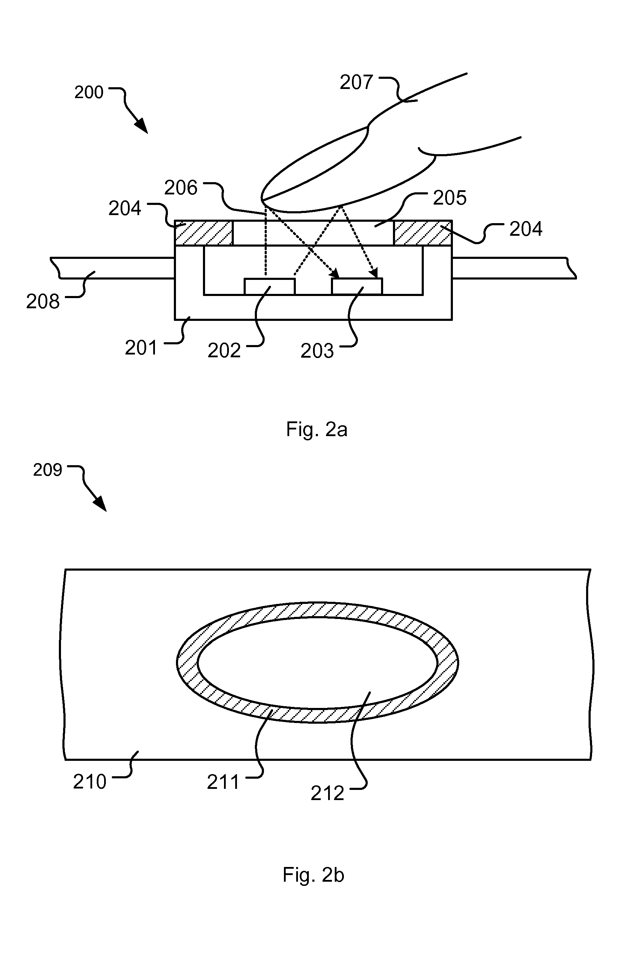

Figure 2a shows an example of a how a sensor 200, in this cases an optical pulse rate sensor, may be realized and integrated into the camera button for monitoring and recording the heart rate of a user. The optical sensor is comprised of a button house 201, housing the sensors and electronics/mechanics needed, and a button surface 204,205 which is the part where the user 207 may touch and operate the button. The button surface 204,205 comprises two surface parts, a non-transparent part 204, and a transparent part 205. The transparency of the transparent part 205 is determined such that it is transparent to the wave lengths used in the measurement while it is non-transparent to other wave lengths. In this way false readings and interference from impinging light having a wave length close to the wave lengths used in the measurement is minimized.

The button house 201 comprise (in this case) a transmitter 202, in this case an infrared (IR) light transmitter, emitting a light 206, in this case IR light, through the transparent part 205 of the button surface 204,205, which in this case is made transparent only to the IR light used by the transmitter 202. The IR light reflects of an object, in this case a finger 207, placed in the vicinity of or on the transparent part 205, onto a detector 203, which in this case is an optical IR detector. The transmitted IR light is preferably modulated with a high frequency in the range 36 - 300kHz which will eliminate potential disturbances from ambient and illumination light. The reflected IR light, coming from the IR transmitter 202 and detected by the IR receiver 203, will vary proportional to the users pulse, and thus the users pulse may be measured by the sensor 200. If another transmitter emitting red light (and a detector capable of detecting the red light) is added to the sensor 200 it will also be possible to measure the blood oxygen level of the user.

The non-transparent part 204, 211 may in a variant be implemented as one of two sensors for measuring the user's heart rate. In this way multiple sensors may be implemented in the same camera button.

The button house 201 may be fastened in the casing 208 and thereby immobilized or it may be movable up and down acting as a regular mechanical button. The camera button 200 is not limited to using IR sensors as described above, on the contrary any type of sensor or sensors which may be fitted into the inner volume of the button house 201 may be used.

Figure 2b shows a view of a camera button 209 in a mobile phone (seen from above). In this example the camera button 209 is elliptical in shape, but the camera button may be made into any practical shape (e.g. circular, rectangular, star shaped, etc.). In figure 2b the button surface 211,212 is shown as two elliptical shapes, an inner transparent part 212 and an outer non-transparent part 211, placed in the casing 210.

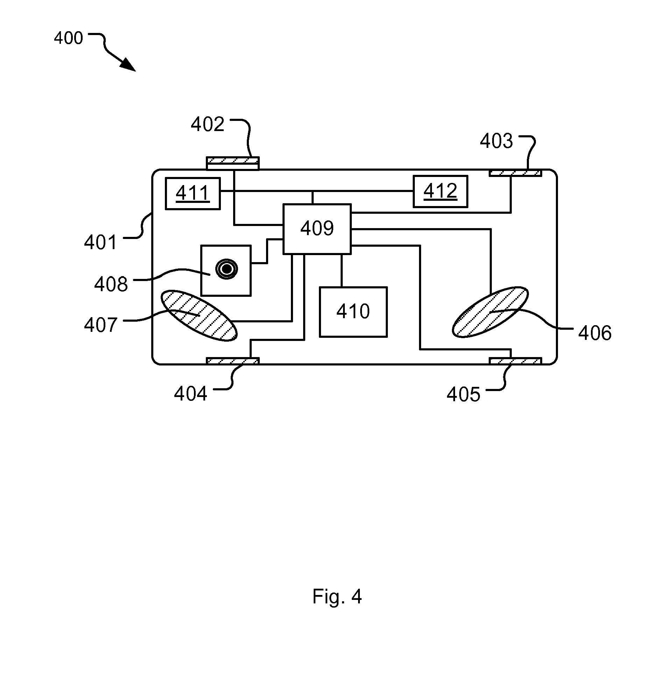

Figure 4 shows a block diagram of a mobile phone 400 according to an embodiment of the present invention. The mobile phone 400 comprise of a camera button with an integrated sensor 402, seven additional sensors 403,404,405,406,407 integrated into the casing 401 of the mobile phone 400, a camera unit 408, processing means 409, a memory 410 and additional two sensors 411,412 (in this case accelerometers) implemented in the interior of the mobile phone. The sensors 402,403,404,405,406,407,411,412 are adapted to record the user's vital signs and provide the recorded sensor information to the processing means. The processing means will, when the camera button is operated, record an image from the camera unit 408 and process the sensor information coming from one or more sensors 402,403,404,405,406,407,411,412 to determine a tag for the image. When a tag has been determined the processing means 409 will assign it to the image and organize and store the image in the memory 410.

In another embodiment other type of sensors (not shown in figure) such as a GPS sensor, ambient temperature sensor, light detector, etc. may also record sensor information in the same manner as the sensors monitoring the user's vital signs 402,403,404,405,406,407. When an image is recorded the other type of sensors will provide their sensor information to the processing means 409, which will include them in the determination of the tag. In this way the tag may be a result of both internal factors relating to the state of the user's body and external factors such as current position.

In a variant the mobile phone may be equipped with more than one camera, for example, for producing three-dimensional images. In this case multiple images may be recorded when the camera button is operated. The multiple images may either be all tagged with the same information or only one (or a number of) image(s) may be tagged. In a variant the different images may be tagged with slightly different information.

In another variant only the sound of an image (for instance when recording a movie) may be stored and tagged using the same process as described above.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" "comprising," "includes" and/or "including" when used herein, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms used herein should be interpreted as having a meaning that is consistent with their meaning in the context of this specification and the relevant art and will not be interpreted in an idealized or overly formal sense unless expressly so defined herein.

The foregoing has described the principles, preferred embodiments and modes of operation of the present invention. However, the invention should be regarded as illustrative rather than restrictive, and not as being limited to the particular embodiments discussed above. The different features of the various embodiments of the invention can be combined in other combinations than those explicitly described. It should therefore be appreciated that variations may be made in those embodiments by those skilled in the art without departing from the scope of the present invention as defined by the following claims.

## Claims

1. A method for tagging a recorded image (300) in a mobile communication device (100), wherein said recorded image is recorded by a camera unit (114) in said mobile communication device (100), the method comprising the steps:
- monitoring (301), using at least one sensor (402,403,404,405,406,407,411,412) in said mobile communication device, a user's vital signs;
- recording sensor information (302) relating to said user's vital signs when said user operates said camera unit (114) in said mobile communication device (100) and is in contact with at least one of said at least one sensors (402,403,404,405,406,407,411,412) in said mobile communication device (100);
- recording an image (303) from said camera unit (114) when said user operates said camera unit (114) in said mobile communication device (100);
- determining a tag (304) based on said recorded sensor information;
- assigning said tag (305) to said recorded image; and
- storing and organizing (306) said recorded image in a memory (410) in said mobile communication device (100) based on said tag.

2. The method according to claim 1, wherein said sensor information comprises information regarding any of the following user's vital signs: body temperature, pulse rate, blood pressure, respiratory rate, blood oxygen level and skin conductance.

3. The method according to any of the previous claims, wherein at least one of said at least one sensor (402,403,404,405,406,407,411,412) is integrated in a camera button (200) in said mobile communication device (100), wherein said at least one sensor (402,403,404,405,406,407,411,412) and said camera button (200) is operated when said user operates said camera unit for recording an image.

4. The method according to any of the previous claims, wherein an image is any of a photograph and a movie.

5. The method according to any of the previous claims, wherein said at least one sensor (402,403,404,405,406,407,411,412) is any of the type: an optical pulse rate sensor, a blood oxygen sensor, an accelerometer, a temperature sensor, and a sensor for measuring electrical resistance.

6. The method according to any of the previous claims, further comprising:
- recording sensor information relating to said user's activity and position from activity sensors and positioning sensors in said mobile communication device (100); and
wherein said determining of said tag is further based on said recorded sensor information relating to said user's activity and position.

7. The method according to any of the previous claims, wherein said at least one sensor (402,403,404,405,406,407,411,412) is placed in a position on the casing (208) of the mobile communication device (100) where said user holds at least one body part (207) when operating said mobile communication device (100), and
- wherein said monitoring, using at least one sensor (402,403,404,405,406,407,411,412), of said user's vital signs are performed via at least one of said at least one body part (207).

8. A mobile communication device (400) adapted for tagging a recorded image, the mobile communication device (400) comprising:
- a camera unit (408) adapted to record an image;
- at least one sensor (402,403,404,405,406,407,411,412) adapted for monitoring a user's vital signs;
- processing means (409) configured to:
- monitoring, using said at least one sensor (402,403,404,405,406,407,411,412), a user's vital signs when said user is in contact with at least one of said at least one sensor (402,403,404,405,406,407,411,412);
- recording sensor information relating to said user's vital signs when said user operates said camera unit (408);
- recording an image from said camera unit when said user operates said camera unit (408) in said mobile communication device;
- determining a tag based on said recorded sensor information;
- assigning said tag to said recorded image; and
- storing means (409) and organizing means (409) adapted to store and to organize said recorded image in a memory (410) in said mobile communication device (400) based on said tag.

9. The mobile communication device (400) according to claim 8, wherein said at least one sensor (402,403,404,405,406,407,411,412) is adapted to monitor sensor information relating to any of the following user's vital signs: body temperature, pulse rate, blood pressure, blood oxygen level, respiratory rate, and skin conductance.

10. The mobile communication device (400) according to any of claims 7 - 8, wherein at least one of said at least one sensor (402,403,404, 405,406,407,411,412) is integrated in a camera button (402) in said mobile communication device (400), wherein said camera button with said integrated sensor (402) is operated when said user operates said camera unit (408) for recording an image.

11. The mobile communication device (400) according to any of claims 7 - 9, wherein said recorded image is any of a photograph and a movie.

12. The mobile communication device (400) according to any of claims 7 - 10, wherein said at least one sensor (402,403,404,405,406,407, 411,412) is any of the type: an optical pulse rate sensor, a temperature sensor, a blood oxygen sensor, an accelerometer, and a sensor for measuring electrical resistance.

13. The mobile communication device (400) according to any of claims 7 - 11, further comprising:
- at least one activity sensor adapted to record sensor information relating to said user's activity;
- at least one positioning sensor adapted to record the position of said mobile communication device (400);
wherein said processing means (409) is further adapted to further determine said tag based on said recorded sensor information relating to said user's activity and to the position of said mobile communication device (400).

14. The mobile communication device (400) according to any of claims 7 - 12, wherein said at least one sensor (402,403,404,405,406, 407,411,412) is placed in a position on the casing (401) of the mobile communication device (400) where said user holds at least one body part (207) when operating said mobile communication device (400), and
- wherein said monitoring, using at least one sensor (402,403,404,405,406,407,411,412), of said user's vital signs are performed via at least one of said at least one body part (207).
